# EUROPEAN PATENT APPLICATION

(11) **EP 4 836 433 A1**
(43) Date of publication of application: **04.11.2026**
(21) Application number: 26175179.6
(22) Date of filing: 28.04.2026
(51) Int. Cl.: H04N 23/67, G02B 7/28, G02B 7/36, G02B 7/38, G03B 13/36, A61B 1/00, G02B 3/14

(54) **SYSTEMS, DEVICES, AND METHODS FOR AUTOMATIC FOCUSING FOR MEDICAL IMAGING**

(30) Priority: 28.04.2025 US 202563796223 P
(71) Applicant: STRYKER CORPORATION, Portage, MI 49002-9711 (US)
(72) Inventor: ANDRÉ, Marc, Portage, 49002 (US); FEINGOLD, Benjamin Hyman, Portage, 49002 (US); WESTWICK, Paul Roald, Portage, 49002 (US)
(74) Representative: V.O.

(57) **Abstract**

A method for automatically focusing a medical camera includes receiving a plurality of images captured using different focal plane settings of the medical camera; while receiving the plurality of images, determining focus metric values for at least some of the plurality of images using a plurality of different focus metrics and setting the different focal plane settings based on the determined focus metric values; determining an optimal focal plane setting based on the focus metric values; and ceasing to set different focal plane settings such that images are captured using the optimal focal plane setting.

## Description

### CROSS-REFERENCE TO RELATED APPLICATIONS

This application claims the benefit of U.S. Provisional Application No. 63/796,223, filed April 28, 2025, the entire contents of which are incorporated herein by reference.

### FIELD

This disclosure relates generally to medical imaging and more specifically to focus adjustment for medical imaging.

### BACKGROUND

Minimally invasive surgery generally involves the use of an endoscopic imaging camera that includes an endoscope inserted through a small incision into a patient to provide a surgeon with a clear and precise view within the body with minimal tissue damage. The endoscope directs light into the surgical cavity, receives light reflected or emitted by tissue within the surgical cavity, and directs the received light to one or more imaging sensors of the camera. Due to the confined nature of the surgical cavity, the field of view provided by an endoscopic imaging camera is often relatively narrow. As such, a surgeon frequently redirects the endoscopic imaging camera as needed to view different regions of tissue. The confined nature of the surgical cavity and the frequent movement of the endoscopic imaging camera often lead to video captured by the endoscopic imaging camera being out of focus. Difficulties with focusing are not limited to endoscopic imaging cameras. Open-field imaging cameras, which are often hand-held, may lose focus as a surgeon directs the camera at different regions of a subject.

Medical imaging cameras, such as endoscopic imaging cameras and open-field imaging cameras, may be configured for manual focus adjustment, enabling a surgeon to adjust the focal plane setting of the camera until the field of view (or desired portion of the field of view) is in focus. However, manual focus adjustment may be cumbersome and time-consuming. Some medical imaging cameras include an automatic focus function that automatically adjusts the focal plane setting of the camera based on assessment of the level of focus of the video. However, automatic focus functions are often relatively slow, which may lead to periods of out-of-focus video that are distracting to the surgeon.

### SUMMARY

According to an aspect, systems, devices, and methods include automatically focusing a medical imager by assessing the focus of captured images using multiple different focus metrics and adjusting a focal plane setting of the medical imager accordingly. The different focus metrics assess contrast in different ways, providing assessments suitable for improving focus both when further away from an optimal focal plane setting and when closer to the optimal focal plane setting. By using multiple different focus metrics, an automatic focusing process can determine an optimal focal plane setting using fewer intermediate focal plane settings relative to a process that utilizes just a single focus metric. By reducing the number of intermediate focal plane settings, automatic focusing can be performed faster, reducing the required amount of computing resources and reducing distraction to a user.

According to an aspect, a method for automatically focusing a medical camera includes receiving a plurality of images captured using different focal plane settings of the medical camera; while receiving the plurality of images, determining focus metric values for at least some of the plurality of images using a plurality of different focus metrics and setting the different focal plane settings based on the determined focus metric values; determining an optimal focal plane setting based on the focus metric values; and ceasing to set different focal plane settings such that images are captured using the optimal focal plane setting.

Determining the focus metric values for at least some of the plurality of images may include determining the focus metric values using the plurality of focus metrics for each image of the at least some of the plurality of images. A focus metric value for a given image may be determined based on a value associated with a first focus metric and a value associated with a second focus metric. Determining the focus metric values for at least some of the plurality of images may include using a first focus metric for a first set of images of the plurality of images and using a second focus metric but not the first focus metric for a second set of images of the plurality of images.

The focus metric values may be determined for at least one region of interest of the plurality of images. The method may include determining the at least one region of interest by analyzing at least one image of the plurality of images using a machine learning model. The method may include displaying at least one image of the plurality of images with an indication of the at least one region of interest. The focus metric values may be determined for multiple regions of interest of the plurality of images.

A first focus metric of the plurality of focus metrics may be associated with more localized contrast than a second focus metric of the plurality of focus metrics. The first focus metric may be associated with a smaller kernel size than the second focus metric.

The method may include displaying a subset of images of the plurality of images, wherein at least one of the focal plane settings is set based on at least one focus metric value determined for at least one image that is not displayed. The method may include switching the focal plane of the medical camera between a focal plane setting used for the subset of images that are displayed and focal plane settings that are set based on focus metric values determined for images that are not displayed.

Optionally, setting the different focal plane settings based on the determined focus metric values includes adjusting the focal plane of the medical camera to a first focal plane setting based on a focus metric value determined for an image of the plurality of images associated with a previously set focal plane setting; prior to determining the focus metric value for an image associated with the first focal plane setting, adjusting the focal plane of the medical camera from the first focal plane setting to a second focal plane setting; and after adjusting the focal plane of the medical camera from the first focal plane setting to the second focal plane setting, determining at least one of the focus metric values for the image associated with the first focal plane setting.

The optimal focal plane setting may be determined in response to a user request to focus the medical camera. Setting the different focal plane settings may include controlling a liquid lens of the medical camera. The method may include receiving a request to focus the medical camera, and in response to receiving the request to focus the medical camera, setting at least one parameter used for determining the optimal focal plane setting to a predetermined value associated with a type of medical procedure. The at least one parameter may include a parameter associated with defining a region of interest, a parameter associated with an initial focal plane setting, or a parameter associated with defining a range of focal plane settings.

Setting the different focal plane settings based on the determined focus metric values may include skipping intermediate focal plane settings when changing from a first focal plane setting to a second focal plane setting. Setting the different focal plane settings based on the determined focus metric values may include changing from the second focal plane setting to one of the intermediate focal plane settings.

According to an aspect, a system includes one or more processors and memory storing one or more programs for execution by the one or more processors, the one or more programs including instruction that when executed by the one or more processors cause the system to: receive a plurality of images captured at different focal plane settings of a medical camera; while receiving the plurality of images, determine focus metric values for at least some of the plurality of images using a plurality of focus metrics and setting the different focal plane settings based on the determined focus metric values; determine an optimal focal plane setting based on the focus metric values; and set a focus of the medical camera to the optimal focal plane setting.

Optionally, the system includes the medical camera.

Determining the focus metric values for at least some of the plurality of images may include determining the focus metric values using the plurality of focus metrics for each image of the at least some of the plurality of images. A focus metric value for a given image may be determined based on a value associated with a first focus metric and a value associated with a second focus metric. Determining the focus metric values for at least some of the plurality of images may include using a first focus metric for a first set of images of the plurality of images and using a second focus metric but not the first focus metric for a second set of images of the plurality of images.

The focus metric values may be determined for at least one region of interest of the plurality of images. The one or more programs may include instructions for determining the at least one region of interest by analyzing at least one image of the plurality of images using a machine learning model. The one or more programs may include instructions for displaying at least one image of the plurality of images with an indication of the at least one region of interest. The focus metric values may be determined for multiple regions of interest of the plurality of images.

A first focus metric of the plurality of focus metrics may be associated with more localized contrast than a second focus metric of the plurality of focus metrics. The first focus metric may be associated with a smaller kernel size than the second focus metric.

The one or more programs may include instructions for displaying a subset of images of the plurality of images, wherein at least one of the focal plane settings is set based on at least one focus metric value determined for at least one image that is not displayed. The one or more programs may include instructions for switching the focal plane of the medical camera between a focal plane setting used for the subset of images that are displayed and focal plane settings that are set based on focus metric values determined for images that are not displayed.

Setting the different focal plane settings based on the determined focus metric values may include adjusting the focal plane of the medical camera to a first focal plane setting based on a focus metric value determined for an image of the plurality of images associated with a previously set focal plane setting; prior to determining the focus metric value for an image associated with the first focal plane setting, adjusting the focal plane of the medical camera from the first focal plane setting to a second focal plane setting; and after adjusting the focal plane of the medical camera from the first focal plane setting to the second focal plane setting, determining at least one of the focus metric values for the image associated with the first focal plane setting.

The optimal focal plane setting may be determined in response to a user request to focus the medical camera. Setting the different focal plane settings may include controlling a liquid lens of the medical camera.

The one or more programs may include instructions for receiving a request to focus the medical camera, and in response to receiving the request to focus the medical camera, setting at least one parameter used for determining the optimal focal plane setting to a predetermined value associated with a type of medical procedure. The at least one parameter may include a parameter associated with defining a region of interest, a parameter associated with an initial focal plane setting, or a parameter associated with defining a range of focal plane settings.

Setting the different focal plane settings based on the determined focus metric values may include skipping intermediate focal plane settings when changing from a first focal plane setting to a second focal plane setting. Setting the different focal plane settings based on the determined focus metric values may include changing from the second focal plane setting to one of the intermediate focal plane settings.

According to an aspect, a method for automatically focusing a medical camera includes adjusting the focal plane of the medical camera to a first focal plane setting based on a focus metric value determined based on an image associated with a previously set focal plane setting; receiving an image captured at the first focal plane setting; adjusting the focal plane of the medical camera from the first focal plane setting to a second focal plane setting; and after adjusting the focal plane of the medical camera from the first focal plane setting to the second focal plane setting, determining at least one focus metric value based on the image captured at the first focal plane setting.

The method may include, prior to adjusting the focal plane of the medical camera from the first focal plane setting to the second focal plane setting, selecting the second focal plane setting based on a prediction associated with an improvement in focus from the previously set focal plane setting to the first focal plane setting. The method may include computing the focus metric values based on a plurality of focus metrics. A first focus metric of the plurality of focus metrics may be associated with more localized contrast than a second focus metric of the plurality of focus metrics. The first focus metric may be associated with a smaller kernel size than the second focus metric.

Adjusting the focal plane may include controlling a liquid lens of the medical camera. The method may include receiving a request to focus the medical camera, and in response to receiving the request to focus the medical camera, setting at least one parameter used for automatically focusing the camera to a predetermined value associated with a type of medical procedure. The at least one parameter may include a parameter associated with defining a region of interest, a parameter associated with an initial focal plane setting, or a parameter associated with defining a range of focal plane settings.

According to an aspect, a system includes one or more processors and memory storing one or more programs for execution by the one or more processors, the one or more programs including instruction that when executed by the one or more processors cause the system to: adjust the focal plane of the medical camera to a first focal plane setting based on a focus metric value determined based on an image associated with a previously set focal plane setting; receive an image captured at the first focal plane setting; adjust the focal plane of the medical camera from the first focal plane setting to a second focal plane setting; and after adjusting the focal plane of the medical camera from the first focal plane setting to the second focal plane setting, determine at least one focus metric value based on the image captured at the first focal plane setting.

The one or more programs may include instructions for, prior to adjusting the focal plane of the medical camera from the first focal plane setting to the second focal plane setting, selecting the second focal plane setting based on a prediction associated with an improvement in focus from the previously set focal plane setting to the first focal plane setting.

The one or more programs may include instructions for computing the focus metric values based on a plurality of focus metrics. A first focus metric of the plurality of focus metrics may be associated with more localized contrast than a second focus metric of the plurality of focus metrics. The first focus metric may be associated with a smaller kernel size than the second focus metric.

Adjusting the focal plane may include controlling a liquid lens of the medical camera. The one or more programs may include instructions for receiving a request to focus the medical camera, and in response to receiving the request to focus the medical camera, setting at least one parameter used for automatically focusing the camera to a predetermined value associated with a type of medical procedure. The at least one parameter may include a parameter associated with defining a region of interest, a parameter associated with an initial focal plane setting, or a parameter associated with defining a range of focal plane settings.

According to an aspect, a computer program product, or a non-transitory computer readable storage medium, comprises, such as stores, instructions for execution by one or more processors of a computing system for causing the computing system to perform any of the methods above.

It will be appreciated that any of the variations, aspects, features, and options described in view of the systems apply equally to the methods and vice versa. It will also be clear that any one or more of the above variations, aspects, features, and options can be combined.

### BRIEF DESCRIPTION OF THE DRAWINGS

The invention will now be described, by way of example only, with reference to the accompanying drawings, in which:
FIG. 1 illustrates an exemplary imaging system for imaging tissue of a subject;
FIG. 2 illustrates an exemplary method that can be performed for automatically focusing a camera;
FIG. 3A illustrates an example in which a focus metric value is determined based on a region of interest of an image and FIG. 3B illustrates an example in which focus metric values are determined for a plurality of different regions of interest of an image;
FIG. 4A is a graph of an exemplary relationship between focus metric values and focal plane settings for images of a given scene captured by a camera, and FIG. 4B illustrates a binary search approach for automatic focusing;
FIG. 5 illustrates an exemplary imaging system that includes a camera having an adjustable lens assembly configured for adjusting a focal plane setting for automatic focusing process;
FIG. 6A illustrates an example of a method for determining a focus metric value based on two focus metrics, and FIG. 6B illustrates an exemplary relationship between focus metric values provided by different focus metrics;
FIG. 7 illustrates an exemplary method for using different focus metrics at different stages of an automatic focusing process;
FIG. 8 illustrates an exemplary method for performing automatic focusing using images that are not displayed;
FIG. 9 illustrates a method for automatic focusing using branch prediction; and
FIG. 10 is a functional block diagram of an exemplary computing system.

### DETAILED DESCRIPTION

In the following description of the various examples, reference is made to the accompanying drawings in which are shown, by way of illustration, specific examples that can be practiced. The description is presented to enable one of ordinary skill in the art to make and use the invention and is provided in the context of a patent application and its requirements. Various modifications to the described examples will be readily apparent to those persons skilled in the art, and the generic principles herein may be applied to other examples. Thus, the present invention is not intended to be limited to the examples shown, but is to be accorded the widest scope consistent with the principles and features described herein.

According to various aspects, systems, devices, and methods described herein perform automatic focusing of a medical camera using multiple different focus metrics to assess the level of focus. Focus metric values are generated for images (e.g., video frames) captured during an automatic focusing process and a focal plane setting is adjusted to improve the focus metric values until an optimal focal plane setting is achieved. The focus metric values are functions of different focus metrics, which assess contrast in different ways. For example, a first focus metric may assess more localized contrast by determining differences in brightness of pixels that are close neighbors, and a second focus metric may assess less localized contrast by determining difference in brightness of pixels that are further away from each other. The second focus metric may provide a stronger signal when the camera is further out of focus than the first focus metric. In contrast, the first focus metric may provide a stronger signal when closer to the optimal focus. By using both focus metrics, an automatic focusing process may achieve the optimal focus more quickly.

Conventional automatic focusing typically includes an approach for assessing focus that provides a strong signal at the optimal focus (e.g., small changes in focal plane setting result in relatively large differences in focus assessment). However, such approaches typically provide a weak signal away from the optimal focus (e.g., small changes in focal plane setting result in little or no difference in focus assessment). Conventional automatic focusing approaches typically sweep across focal plane settings from one end of a range of focal plane settings toward the other end, suing relatively small focal plane adjustments, until the peak is located. With this incremental sweeping approach, the peak will eventually be found despite the focus assessment signal being relatively weak further away from the optimal focus does. However, the incremental sweeping approach is relatively slow, which can prove distracting to a viewer as the viewer waits for the automatic focusing process to find the optimal focus.

In contrast to the conventional approach, the automatic focusing processes described herein provide faster automatic focusing by using multiple focus metrics that, in combination, provide strong signals both close to the optimal focus and further away from the optimal focus. By using multiple focus metrics, automatic focusing processes according to the principles described herein are not limited to conventional incremental sweeping techniques for finding the optimal focal plane setting. Rather, the automatic focusing processes can make relatively large jumps in focal plane settings, resulting in using fewer focal plane settings to find the optimal focal plane setting as compared to a conventional incremental sweeping approach.

A medical camera for which the automatic focusing processes described herein may be used may include a liquid lens, which provides a faster response time and greater number focal plane settings than mechanical focal plane adjustment systems. The automatic focusing processes described herein may be particularly advantageous for cameras with liquid lenses since a conventional incremental sweeping approach that utilizes the greater number of focal plane settings available with liquid lenses may require a relatively long time to locate the optimal focal plane setting. In contrast, the automatic focusing processes described herein, which are not limited to incremental sweeping, can take advantage of the fast response times and greater number of focal plane settings provided by liquid lenses while providing faster automatic focusing than conventional approaches.

As noted above, a focus metric may be configured to assess less localized contrast of an image. Implementation of such a focus metric may include downscaling an image and applying a contrast assessment function, such as a Laplacian derivative, to the downscaled image. Alternatively, implementation of the focus metric may include computing the contrast of a given pixel based on pixels that are further away from the given pixel than its nearest neighbors.

Multiple focus metrics may be used to assess contrast of a given image. For example, a first focus metric value that is determined based on a first focus metric and a second focus metric values that is determined based on a second focus metric may be determined for the same image. The different focus metric values may be combined into a focus metric value that is a function of both focus metrics.

Different focus metrics may be used at different times of an automatic focusing process. For example, a first focus metric configured to provide a stronger signal when further away from an optimal focal plane setting may be used in a first stage of an automatic focusing process and a second focus metric configured to provide a stronger signal when closer to an optimal focus plane setting may be used in a second stage of an automatic focusing process.

By using multiple different focus metrics, automatic focusing processes, according to the principles described herein, can determine an optimal focal plane setting using a fewer number of focal plane settings relative to a process that utilizes just a single focus metric. By reducing the number of focal plane settings, automatic focusing can be performed faster, reducing the required amount of computing resources and reducing distraction to a user.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units, but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like refer to the action and processes of a computer system, or similar electronic computing device, that manipulates and transforms data represented as physical (electronic) quantities within the computer system memories or registers or other such information storage, transmission, or display devices.

The present disclosure in some aspects also relates to devices or systems for performing the operations herein. The devices or systems may be specially constructed for the required purposes, may comprise a general-purpose computer selectively activated or reconfigured by a computer program stored in the computer, or may include any combination thereof. Computer instructions for performing the operations herein can be stored in any combination of non-transitory, computer-readable storage medium, such as, but not limited to, any type of disk, including USB flash drives, external hard drives, optical disks, CD-ROMs, magnetic-optical disks, read-only memories (ROMs), random access memories (RAMs), EPROMs, EEPROMs, magnetic or optical cards, or any type of media suitable for storing electronic instructions, and each coupled to a computer system bus. One or more instructions for performing the operations herein may be implemented in or executed by one or more Application Specific Integrated Circuits (ASICs), Field Programmable Gate Arrays (FPGAs), Digital Signal Processing units (DSPs), Graphics Processing Units (GPUs), Central Processing Units (CPUs), or any other suitable processing unit. Furthermore, the computers referred to herein may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

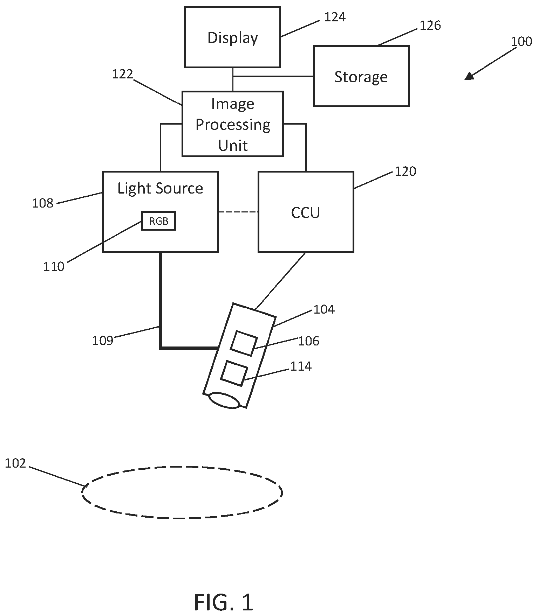

FIG. 1 illustrates an exemplary imaging system 100 for imaging a target region 102 of tissue of a subject during an imaging session, which can be an imaging session occurring during a surgical procedure or a non-surgical medical procedure. The system 100 includes a medical camera 104 (also referred to herein as a camera for simplicity). The camera 104 has at least one image sensor 106 configured to capture images (e.g., single snapshot images and/or a series of video frames) of a target region. (The terms "image" and "frame" are used interchangeably throughout.) The camera 104 may include at least one adjustable lens 114 for adjusting a focal plane setting of the camera 104. The at least one adjustable lens 114 may include an electronically controlled adjustable lens 114 that may be controlled electronically to adjust the focal plane setting of the camera 104. The at least one adjustable lens 114 may be manually adjustable (in addition to being electronically adjustable) or may include a combination of at least one manually adjustable lens and at least one non-manually adjustable lens. The camera 104 can be a hand-held device, such as a hand-held open-field camera or a hand-held endoscopic camera, or can be a device mounted or attached to a mechanical support arm (e.g., a robotic or non-robotic arm).

The camera 104 may be connected to a camera control unit (CCU) 120, which may generate one or more single snapshot images and/or video frames (referred to herein collectively as "images") from imaging data generated by the camera 104. The CCU 120 may send control signals to the camera 104, such as to control the adjustable lens 114 to achieve different focal plane settings, to control a mechanical shutter of the camera 104, and/or to adjust a gain of the at least one image sensor 106. In some variations, the functionalities of the CCU 120 and the camera 104 are incorporated into a single device.

The images generated by the CCU 120 may be transmitted to an image processing unit 122 that may apply one or more image processing techniques described further below to the images generated by the CCU 120. Optionally, the functionalities of the CCU 120 and the image processing unit 122 described herein are integrated into a single device. The image processing unit 122 (and/or the CCU 120) may be connected to one or more displays 124 for displaying the one or more images generated by the CCU 120 or one or more images or other visualizations generated based on the images generated by the CCU 120. The image processing unit 122 (and/or the CCU 120) may store the one or more images generated by the CCU 120, or one or more images or other visualizations generated based on the images generated by the CCU 120, in one or more storage devices 126. The one or more storage devices 126 can include one or more local memories, one or more remote memories, a recorder or other data storage device, a printer, and/or a picture archiving and communication system (PACS). The system 100 may additionally or alternatively include any suitable systems for communicating and/or storing images and image-related data.

The images generated using CCU 120 may be described herein as visible (e.g., white) light images. However, it is to be understood that the imaging system 100 of FIG. 1 may be configured to generate images and/or video frames according to any other suitable imaging modality, including, but not limited to, fluorescence imaging, radiation imaging (e.g., X-ray images gathered from imaging procedures such as CT scans, PET scans, etc.), MRI, and/or ultrasound imaging. Thus, the image processing unit 122 may be configured to process medical images of various modalities, such as visible light images, fluorescence images, X-ray images, MRI images, ultrasound images, etc.

The imaging system 100 may include a light source 108 configured to generate light that is directed to the field of view to illuminate the target region 102. Light generated by the light source 108 can be provided to the camera 104 by a light cable 109. The camera 104 may include one or more optical components, such as one or more lenses, fiber optics, light pipes, etc., for directing the light received from the light source 108 to the tissue. The camera 104 may be an endoscopic camera that includes an endoscope that includes one or more optical components for conveying the light to a scene within a surgical cavity into which the endoscope is inserted. The endoscope may be pre-inserted, i.e. inserted into the cavity prior to the start of any method described herein. The camera 104 may be an open-field camera and may include one or more lenses that direct the light toward the field of view of the open-field imager.

The light source 108 includes one or more visible light emitters 110 that emit visible light in one or more visible wavebands (e.g., full spectrum visible light, narrow band visible light, or other portions of the visible light spectrum). The visible light emitters 110 may include one or more solid state emitters, such as LEDs and/or laser diodes, and/or one or more non-solid state emitters. The visible light emitters 110 may include red, green, and blue (or other color component) LEDs or laser diodes that in combination generate white light or other illumination needed for reflected light imaging. These color component light emitters may be centered around the same wavelengths around which the camera 104 is centered. For example, in variations in which the camera 104 includes a single-chip, single-color image sensor having an RGB color filter array deposited on its pixels, the red, green, and blue light sources may be centered around the same wavelengths around which the RGB color filter array is centered. As another example, in variations in which the camera 104 includes a three-chip, three-sensor (RGB) color camera system, the red, green, and blue light sources may be centered around the same wavelengths around which the red, green, and blue image sensors are centered.

The light source 108 may include one or more optical elements that shape and/or guide the light output from the visible light emitters 110. The optical components may include one or more lenses, mirrors (e.g., dichroic mirrors), light guides, and/or diffractive elements, e.g., to help ensure a flat field over substantially the entire field of view of the camera 104.

The camera 104 may acquire reflected light images from visible light reflected from the tissue that is incident on the at least one image sensor 106. The at least one image sensor 106 may include at least one solid state image sensor. The at least one image sensor 106 may include, for example, a charge-coupled device (CCD), a CMOS sensor, a CID, or other suitable sensor technology. The at least one image sensor 106 may include a single image sensor (e.g., a grayscale image sensor or a color image sensor having an RGB color filter array deposited on its pixels). The at least one image sensor 106 may include multiple sensors, such as a sensor for detecting red light, a sensor for detecting green light, and a sensor for detecting blue light. Any number of sensors may be used, including two sensors or four sensors.

Imaging system 100 may be configured to automatically focus the camera 104. For example, CCU 120 may analyze images captured by camera 104 to assess the degree of focus of the images and may control adjustment of a lens system of the camera 104 until the images are sufficiently in focus. Imaging system 100 may perform automatic focusing of the camera 104 continuously or may perform automatic focusing of the camera 104 only when in an automatic focusing mode. Imaging system 100 may switch to an automatic focusing mode in response to a user input. For example, a user may request automatic focusing using a suitable input device, such as a button press on camera 104 or a voice command. In response to the user request for automatic focusing, imaging system 100 may switch to an automatic focusing mode in which the imaging system 100 (e.g., CCU 120 and camera 104) automatically adjusts the lens system of the camera 104 until images captured by the camera 104 are sufficiently in focus. Optionally, imaging system 100 may automatically switch out of an automatic focusing mode once imaging system 100 has determined that camera 104 is in focus.

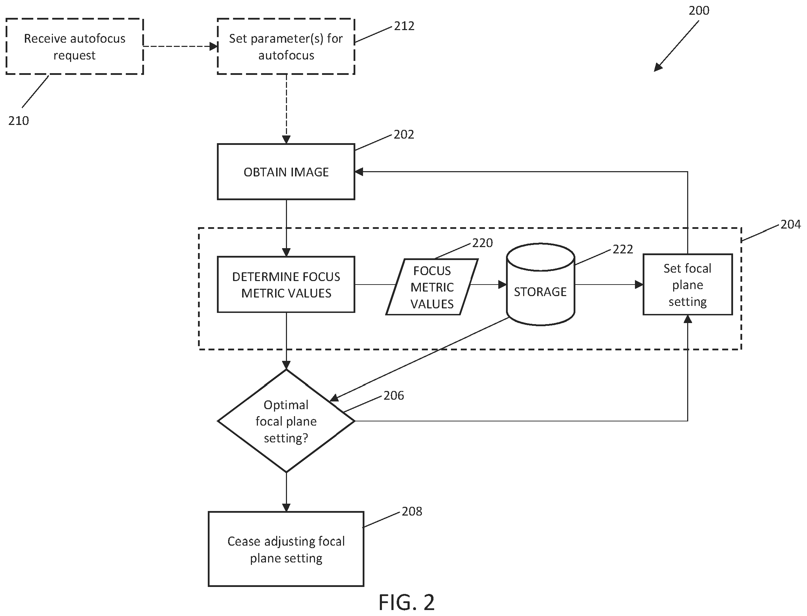

FIG. 2 illustrates a method 200 that can be performed by imaging system 100 for automatically focusing the camera 104, such as in an automatic focusing mode. One or more steps of method 200 may be performed, for example, by CCU 120 and/or image processing unit 122 of FIG. 1.

At step 202, a plurality of images captured using different focal plane settings of a medical camera are received. The plurality of images may be received at one or more processors configured to determine focus metrics for the plurality of images and set focal plane settings based on the focus metrics. For example, with reference to FIG. 1, video frames captured by the camera 104 using different focal plane settings may be received by one or more processors of CCU 120. Alternatively, a medical imager may include one or more processors configured to determine focus metrics and set focal plane settings, and the one or more processors may receive the plurality of images from one or more image forming processors of the medical camera. The plurality of images received at step 202 may be a sequence of video frames.

At step 204, focus metric values are determined for at least some of the plurality of images using a plurality of different focus metrics. The focus metric values are determined by the one or more processors that received the images in step 202. For example, with reference to FIG. 1, focus metric values may be determined by one or more processors of CCU 120. Focus metric values may be determined for each of the images received at step 202. For example, the images received at step 202 may be a sequence of video frames, and at least one focus metric value may be determined for each of the frames in the sequence of video frames. Alternatively, focus metric values may be determined for a subset of the images received at step 202. For example, a sequence of video frames may be sampled, and focus metric values may be determined for the sampled frames. The sequences of video frames may be sampled at a fraction of a frame rate of the video frames, such as half the frame rate, a third of the frame rate, a quarter of the frame rate, a tenth of the frame rate, etc.

Determining focus metric values according to step 204 includes, for a given image, using at least one focus metric to determine at least one focus metric value based on image data from the given image. In general, the focus metric value is an estimate of contrast associated with the image data, and a focus metric is the manner in which (e.g., with one or more calculations or processing steps) image data is processed to generate the estimate of the contrast associated with the image data. Different focus metrics assess contrast in different ways, providing assessments suitable for improving focus both when further away from an optimal focal plane setting and when closer to the optimal focal plane setting. By using multiple different focus metrics, an automatic focusing process can determine an optimal focal plane setting using fewer intermediate focal plane settings relative to a process that utilizes just a single focus metric. By reducing the number of intermediate focal plane settings, automatic focusing can be performed faster, reducing the required amount of computing resources and reducing distraction to a user.

Different focus metrics may differ based on how they compute an estimate of contrast for a given location (e.g., pixel) of an image. One focus metric may compute estimates of contrast for a given pixel of an image based on differences in brightness between the pixel and "close" neighbors-pixels within a relatively small region around the given pixel (e.g., its nearest neighbors). Another focus metric may compute estimates of contrast for a given pixel based on differences in brightness with pixels further away than the close neighbors (e.g., further away than the nearest neighbors) of the given pixel. A focus metric that computes contrast based on nearest neighbors (or other close neighbors) may provide a more localized assessment of contrast than a focus metric that computes contrast based on pixels further away. A less localized focus metric may be more suitable when an image is more out of focus since changes in focal plane settings may have more effect on focus at a macro level when further from an optimal focus. Thus, a less localized focus metric may provide a stronger signal (small changes in focal plane setting provide a greater change in focus metric value) than a more localized focus metric when a camera is more out of focus. In contrast, a more localized focus metric may better assess local contrast, which may provide higher levels of focus than a less localized focus metric. In other words, a more localized focus metric may provide a stronger signal when a camera is closer to an optimal focus.

Focus metric may be defined that compare a locally smoothed pixel to locally smoothed pixels at a variety of distances. A locally smoothed pixel is a value that is a function of the given pixel value and nearby pixel values. Locally smoothed pixel values may be computed by applying a suitable smoothing function to an image or portion of an image. In general, various focus metrics may differ from one another in two degrees of freedom-how smoothed the central value (the "given pixel") is in the metric and how large of an area is taken into account when comparing that central value to other values.

A single focus metric may be used to determine a focus metric value for a given image. Different focus metrics may be used to determine focus metric values for different images. For example, a first focus metric may be used during an earlier stage of an automatic focusing process and a second focus metric may be used during a later stage of the automatic focusing process. Alternatively, multiple focus metrics may be used to determine a focus metric value for a given image. For example, a focus metric value for an image may be a combination of intermediate focus metric values determined using multiple focus metrics. For example, a focus metric value for an image may be determined by combining (e.g., using an average, a weighted average, a sum, etc.) a first intermediate focus metric value determined based on a first focus metric and a second intermediate focus metric value determined based on a second focus metric.

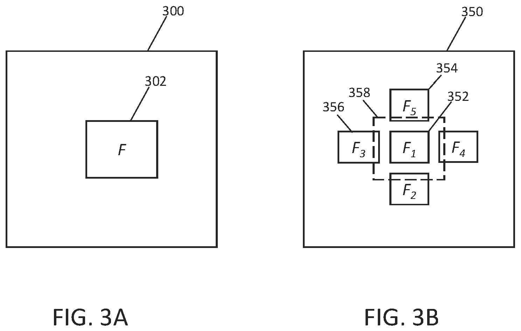

A single focus metric value may be determined for a given image. The single focus metric value may be determined based on all pixels of the image, or the single focus metric value may be determined based on less than all of the pixels of the image, such as based on a region of interest of the image (e.g., a central region of pixels of an image). FIG. 3A illustrates an example in which a focus metric value *F* is determined based on a region of interest 302 of an image 300, which in the illustrated example is a central region of the image 300. Alternatively, multiple focus metric values may be determined for a given image. For example, a focus metric value may be determined for each of a plurality of different regions of interest of the image. FIG. 3B illustrates an example in which focus metric values are determined for a plurality of different regions of interest (e.g., region 352, region 354, region 356) of an image 350. Focus metric values may be determined for regions of interest that are different sizes, which may overlap. For example, a focus metric value may be determined for region of interest 358 and region of interest 352, with region of interest 358 being larger than region of interest 352 but centered at the same location. A focus metric value for a larger region of interest 358 may be optimized for ignoring transient high-contrast features, such as debris and bubbles, whereas a focus value for a smaller region of interest 352 may be better at optimizing for deep cavity work. As such, using focus metric values for both the larger region of interest 358 and the smaller region of interest 352 may provide a balance between these optimizations.

In some examples, the region of interest or regions of interest for which focus metric values are calculated are predetermined. For example, region 302 of FIG. 3A may be a region of NxN pixels centered within each image 300. In other examples, the region of interest is determined dynamically, such as for each image. With reference to FIG. 1, a region of interest may be determined dynamically by image processing unit 122. Image processing unit 122 may analyze an image using one or more machine learning models to identify a region of interest within the image that is suitable for determining focus metric values. The one or more machine learning models may be trained to identify features in an image that may be suitable for determining focus, such as tissue, and/or may be trained to identify features that are not suitable for determining focus, such as bubbles within liquid, debris, moisture, or surgical tools. A region of interest may be determined based on the region of interest having the features that are suitable for determining focus on and/or not having the features that are not suitable for determining focus. A region of interest may be determined using non-machine learning approaches, such as by segmenting an image based on color or brightness. For example, brightness values of an image may be determined and a region of the image that includes the brightest regions may be selected as the region of interest. A region of interest may be dynamically determined based on a given image, and the dynamically determined region of interest may be used for one or more subsequent images. As such, a dynamically determined region of interest can be used for multiple images and need not be determined for each of the multiple images (though it may be).

Optionally, a region of interest for which one or more focus metrics are determined may be indicated to a user. For example, with reference to FIG. 3A, an outline indicating region of interest 302 may be overlaid on a display of image 300, such as displayed on display 124 of FIG. 1.

Step 204 also includes setting the different focal plane settings of the camera used to capture the images received at step 202, with the different focal plane settings being set based on the determined focus metric values. As such, steps 202 and 204 are performed in parallel. With reference to FIG. 1, setting the different focal plane settings of the camera may include CCU 120 sending a command to camera 104 to adjust a focal plane setting (e.g., adjust the lens system to achieve a different focal plane) of the camera 104.

In general, the focal plane settings are set using a search algorithm that seeks to maximize focus metric values. A focal plane setting may be set based on a comparison between a focus metric value for an image N and a focus metric value for one or more images captured prior to image N. For example, if a focus metric value for image N captured using a focal plane setting X is greater than a focus metric value for a previously captured image N-1 that was captured using a focal plane setting X-1, then a focal plane setting may be set to a focal plane setting X+1 for capturing a next image N+1. If, however, the focus metric value for image N is less than the focus metric value for image N-1, then the focal plane setting may be set to a focal plane setting X-2.

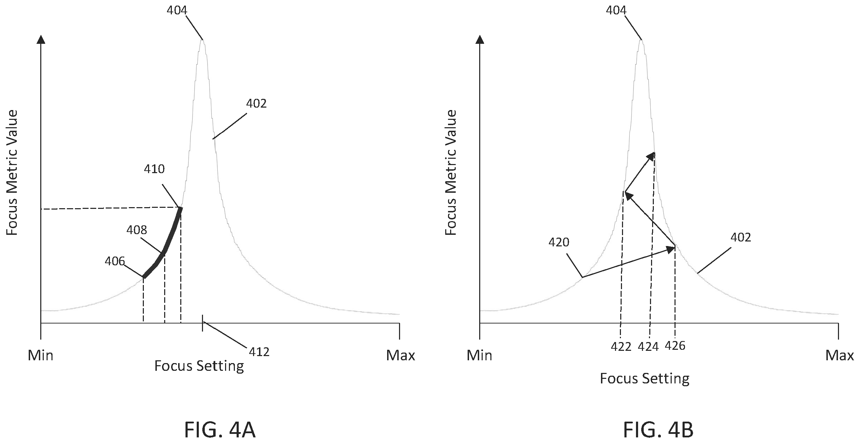

FIG. 4A is a graph of an exemplary relationship between focus metric values and focal plane settings for images of a given scene captured by a camera, such as camera 104 of FIG. 1. Curve 402 represents the focus metric value for a given focal plane setting. Step 204 as applied to graph 400 seeks to adjust the focal plane setting to achieve the maximum focus metric value 404. Assuming for the sake of illustration that images have been captured corresponding to points 406, 408, and 410 of curve 402, step 204 may include using a sweep algorithm that results in selection of focal plane setting 412 as a next focal plane setting based on the trend of increasing focus metric values from point 406 to point 410. Setting a focal plane to focal plane setting 412 may be based on determining from the focus metric values associated with points 406, 408, and 410 that the focus metric value associated with point 410 has increased relative to the focus metric values associated with previously captured images associated with points 406 and 408. The focal plane setting may be increased until the focus metric value peaks or begins to decrease. The focal plane settings may be adjusted from image to image in a linear fashion according to a predetermined increment. The focal plane setting may be adjusted from image to image based on a rate of change of the focus metric value. For example, greater increments of focal plane setting changes from one image to the next may be used for lower rates of change of the focus metric value associated with the flatter regions of curve 402, and lesser increments of focal plane setting changes from one image to the next may be used for higher rate of change of the focus metric value associated with the peaking region of curve 402.

The example focal plane setting method described above is merely intended to illustrate the general approach of step 204. Any suitable search algorithm may be used to set focal plane settings to achieve focus. In some examples, focal plane settings are adjusted in linear fashion, such as using a sweeping algorithm. For example, if a trend of focus metric values indicates that a focal plane setting should be increased from a current focal plane setting, the next focal plane setting may be a predetermined increment higher than the current focal plane setting. The predetermined increment may be the minimum increment achievable by the camera or may be any desired multiple of a minimum increment achievable by the camera.

In some examples, setting focal plane settings according to step 204 includes making relatively large jumps in focal plane settings that include skipping over intermediate focal plane settings. Focal plane settings that have been skipped over may or may not be returned to. For example, with reference to FIG. 4B, which illustrates a binary search approach for automatic focusing, starting from the focal plane setting associated with point 420 of curve 402, the next focal plane setting may be set to focal plane setting 426, skipping over focal plane setting 422 and focal plane setting 424. Since the focus metric value associated with focal plane setting 424, the next focal plane setting may be set to focal plane setting 422, which was previously skipped over. Since the focus metric value associated with focal plane setting 422 is greater than the focus metric value associated with focal plane setting 424, the next focal plane setting may be focal plane setting 424, which had been skipped over twice. As such, step 204 may include jumping back and forth (e.g., on either side of the maximum focus metric value 404) until the maximum focus metric value 404 is achieved. An exemplary algorithm that may include jumping back and forth within a range of focal plane settings that may be used in step 204 is a golden section search algorithm, which may tend to reach the maximum focus metric value 404 faster (i.e., with fewer focal plane settings) than a sweep algorithm.

Returning to FIG. 2, step 204 may include storing focus metric values 220 in memory 222 (e.g., in memory of CCU 120) for use in determining a focal plane setting adjustment. As such, a given focal plane setting adjustment may be determined in step 204 based on focus metric values 220 for one or more previously captured images that are obtained from the memory 222. A next focal plane setting may be determined based on a focus metric value 220 for an image captured using a current focal plane setting (alone or in combination with focus metric values 220 for one or more images captured using previous focal plane settings). In some examples, a next focal plane setting is determined based on focus metric values 220 for one or more images captured using previous focal plane settings and not based on a focus metric value determined for a current focal plane setting, which may reduce latency.

At step 206, an optimal focal plane setting is determined based on the focus metric values determined in step 204. Step 206 may include determining that a focus metric value determined from an image captured at a given focal plane setting is a maximum focus metric value based on focus metric values determined for one or more previously captured images. In general, step 206 is the culmination of the search algorithm used in step 204. The optimal focal plane setting may be associated with a maximum focus metric obtained during step 204. Step 206 may include determining the maximum focus metric value (e.g., maximum focus metric value 404 of curve 402 of FIG. 4) or may include determining a focus metric value that is within a threshold amount of a maximum possible focus metric value that is achievable. For example, the optimal focal plane setting determined at step 206 may be a focal plane setting associated with an image that provides a focus metric value that is sufficiently close to a maximum possible focus metric value for the given circumstances. In other words, the optimal focal plane setting need not be a focal plane setting that achieves the maximum focus metric value but can be one that is close enough to (e.g., within a threshold amount of) the focal plane setting that achieves the maximum focus metric value.

At step 208, adjusting the focal plane settings of the camera ceases such that the optimal focal plane setting is maintained and subsequent images are captured using the optimal focal plane setting. Step 208 may be the end of the automatic focusing mode. For example, CCU 120 may switch from an autofocusing mode to a standard mode in which the focal plane setting is not automatically adjusted. Alternatively, wherein automatic focusing is performed continuously, the optical focal plane setting may be maintained until a focus metric value for a given image fails to meet a predetermined threshold.

As noted above, step 204 includes setting different focal plane settings of a camera. With reference to FIG. 1, this can include the CCU 120 controlling camera 104 to adjust the focal plane settings while capturing the plurality of video frames. The camera 104 may include an adjustable lens system that enables a focal plane setting to be adjusted such that the video frames can be captured with different focal plane settings.

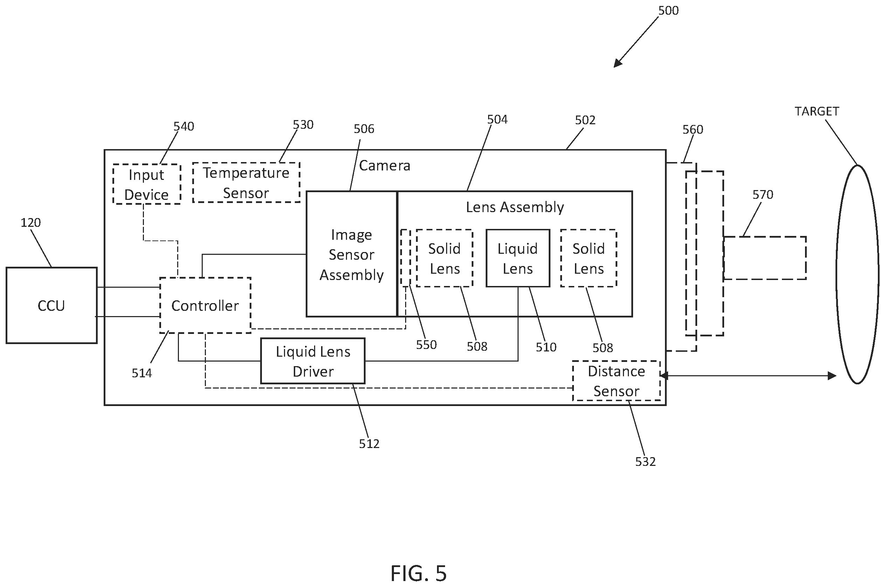

FIG. 5 illustrates an exemplary imaging system 500 that includes a camera 502 having an adjustable lens assembly 504 configured for adjusting a focal plane setting for an automatic focusing process. Camera 502 may be used for camera 104 of system 100. Camera 502 may be an endoscopic imaging camera (which may include a coupler 560 for coupling to an endoscope 570), an open-field imaging camera, or any other type of medical imaging camera. Adjustable lens assembly 504 is positioned in front of an image sensor assembly 506 for focusing light onto the image sensor assembly 506 and may include at least one solid lens 508 (e.g., at least one glass lens) and at least one liquid lens 510. Power applied to the liquid lens 510 can be controlled (e.g., via voltage control or current control) to control a radius of curvature of the liquid lens, thereby controlling a focal plane setting of adjustable lens assembly 504. The one or more solid lenses 508 and the liquid lens 510 can be arranged in any suitable manner. For example, one or more solid lenses 508 may be positioned proximally of the liquid lens 510 (i.e., between the liquid lens 510 and the image sensor assembly 506) and/or one or more solid lenses 508 may be positioned distally of the liquid lens 510. Optionally, the liquid lens 510 may be spaced a sufficient distance away from the image sensor assembly 506 to mitigate heat from the assembly from heating the liquid lens enough to substantially affect focus performance. Adjustable lens assembly 504 may include an adjustable aperture 550.

The camera may include a liquid lens driver 512 that provides a controlled power to the liquid lens 510. The liquid lens driver 512 may receive control commands from CCU 120 (directly or via a controller 514 of the camera 502) that control the focal plane setting of the liquid lens 510. For example, at step 204 of method 200 of FIG. 2, CCU 120 may transmit a command to liquid lens driver 512 to set a desired focal plane setting. Alternatively, the controller 514 can send control commands to the liquid lens driver 512 to set a desired focal plane setting. The controller 514 may receive an instruction from the CCU 120 to set the desired focal plane setting. In some examples, the functionality of CCU 120 is incorporated into the controller 514, and controller 514 determines a focal plane setting and controls the liquid lens driver 512 to set the determined focal plane setting. For example, controller 514 may perform one or more steps of method 200 of FIG. 2, such as steps 202 to 208.

The automatic focusing processes described herein may be particularly advantageous for use with the liquid lens 510 of camera 502 since the liquid lens 510 provides a faster response time and greater number of focal plane settings than mechanical focal plane setting systems. Automatic focusing processes described herein that use a binary search approach (as discussed above with respect to FIG. 4B) are not limited in speed by the greater number of focal plane settings and, thus, the large number of focal plane settings provided by the liquid lens 510 does not limit the speed of the automatic focusing (as it may with incremental sweep approaches to automatic focusing).

The power provided to the liquid lens 510 may be adjusted for purposes other than changing the focal plane setting of the liquid lens 510. For example, the power provided to the liquid lens 510 may be adjusted to account for changes in the temperature of the camera 502 determined, for example, based on reading from a temperature sensor 530. Optionally, the controller 514 may control the adjustable aperture 550.

The focal plane setting of the liquid lens 510 may be changed for each frame of a series of video frames or may be changed at a lower rate such that multiple frames are captured with a given focal plane setting. For example, where the video frames are generated at a frame rate, the focal plane setting may be changed at a rate of half the frame rate, a third of the frame rate, a quarter of the frame rate, a tenth of the frame rate, or any other fraction of the frame rate. A change from one focal plane setting to another may occur when the image sensor(s) is not collecting light from the scene, such as when a shutter of the camera is blocking light from the scene or when no illumination light is being provided to the scene.

Adjustable lens assembly 504 may include a motorized lens assembly configured for high-speed focal plane setting adjustment that is sufficient for generating extended depth of field video frames, additionally or alternatively to liquid lens 510. The motorized lens assembly may include, for example, an electric motor, a magnetic motor, or an ultrasonic motor.

The liquid lens assembly 504 is capable of being set within a range of focal plane settings that extend from a first focal plane setting limit to a second focal plane setting limit. An automatic focusing process, such as method 200 of FIG. 2, may start at or near the first focal plane setting limit or at or near the second focal plane setting limit and adjust the focal plane setting until the optimal focal plane setting is determined. To the extent that the liquid lens assembly 504 is not set at the first focal plane setting limit or the second focal plane setting limit, the start of an automatic focusing process may include setting the liquid lens assembly 504 to the first focal plane setting limit or the second focal plane setting limit. Alternatively, an automatic focusing process may begin with whatever focal plane setting the liquid lens assembly 504 is in when the automatic focusing process is initiated.

Optionally, a focal plane setting range used for an automatic focusing process, such as method 200 of FIG. 2, may be controlled based on a working distance (a distance between the camera 502 and the target region of the subject). For example, a first range of focal plane settings that are suitable for a working distance or range of working distances may be used when the camera 502 is being used at that working distance or range of working distances, whereas a second range of focal plane settings that are suitable for another working distance or range of working distances may be used when the camera 502 is being used at the other working distance or range of working distances. The working distance at which the camera 502 is being used, and thereby the range of focal plane settings used for automatic focusing at the working distance, may be determined based on at least one distance measurement associated with a distance between the camera 502 and the target region of the subject. The at least one distance measurement may be generated by at least one distance sensor 532 of the camera 502. Optionally, multiple distance sensors 532 may be used to obtain distance measurements for multiple different locations of the target region. Additionally, or alternatively, a working distance may be determined based on processing one or more images to determine depth in the images. For example, image processing unit 122 may analyze images (e.g., using a machine learning model) to estimate a depth in one or more regions of an image. The depth estimate(s) may be used by image processing unit 122 or CCU 120 to select a range of focal plane settings to be used by CCU 120 in an automatic focusing process.

Optionally, a range of focal plane settings may be selected based one or more characteristics of operation of the imaging system 500 associated with brightness. The brightness of the scene may correlate with the working distance and, as such, one or more characteristics of operation of the imaging system 500 associated with the brightness of the scene may be used as a kind of proxy for working distance to select the range of focal plane settings. An exemplary characteristic that may be used as a proxy for working distance is a brightness level of a light source (e.g., light source 108). The imaging system 500 (e.g., CCU 120) may automatically determine a brightness of a scene, such as via image analysis, and may automatically set a brightness level of the light source based on the brightness of the scene. Different focal plane setting ranges may be associated with different light source brightness levels, and the focal plane setting range associated with a given light source brightness level may be selected. Other exemplary characteristics that may be used as a proxy for working distance in selecting the range of focal plane settings include image sensor gain and exposure time, each of which may be automatically set by CCU 120 based on image analysis.

A focal plane setting range used for an automatic focusing process, such as method 200 of FIG. 2, may be controlled based on a type of procedure (e.g., surgical specialty). Different types of procedures may be associated with different ranges of working distances. For example, laparoscopic procedures may be associated with longer working distances than arthroscopic procedures. As such, CCU 120 may set focal plane settings within a range of focal plane settings associated with a particular procedure type for which the camera 502 is being used. The procedure type may be set by a user, such as via an input to image processing unit 122, which provides an associated instruction to CCU 120. Alternatively, the procedure type may be determined based on image processing performed by image processing unit 122. For example, image processing unit 122 may use one or more machine learning models to detect one or more features in one or more images that are associated with a particular procedure type and may determine the procedure type based on the one or more features detected in the images.

The set of focal plane settings that are used in an automatic focusing process may depend on a previous automatic focusing process. For example, CCU 120 may respond to a user request to focus that occurs within a threshold amount of time after the completion of an automatic focusing process (e.g., indicating that the previous automatic focusing process was not successful) by searching a different set of focal plane settings than used in the previous automatic focusing process. This may include expanding a range of focal plane settings used in the automatic focusing process or searching a different portion of a range of focal plane settings than searched in a previous automatic focusing process.

Optionally, an initial focal plane setting in an automatic focusing process is associated with a user-set focus. For example, camera 502 may include an input device 540 (e.g., a button, a slider, a knob, a rotatable ring, a touch input device, etc.) that a user may use to set a focus of the camera 502, and an automatic focusing process may set an initial focal plane setting based on the user-set focus.

The CCU 120 may implement different modes for automatic focusing, such as a continuous focusing mode and a trigger focus mode. The continuous focusing mode may include the CCU 120 continuously executing the automatic focusing process (e.g., in real time) to keep the video in focus. In the trigger focus mode, the CCU 120 may use the automatic focusing process in response to a user request to focus (e.g., a user press of a button of the camera 104). As such, method 200 of FIG. 2 may include an optional step 210 of the CCU 120 receiving a request to automatically focus the camera (e.g., a user may provide an input to the camera or to any other system component communicatively connected to the CCU 120). The CCU 120 may enter an automatic focusing mode in response to the request.

Method 200 may include an optional step 212 of setting a value of one or more parameters used for automatic focusing. The one or more parameters that are set may include a parameter associated with defining a range of focal plane settings (e.g., a range associated with a measured or predicted working distance), such as a first focal plane setting limit or a second focal plane setting limit. The one or more parameters may include a parameter associated with setting an initial focal plane setting (e.g., a value associated with a measured or predicted working distance). The one or more parameters may include a parameter associated with defining a region of interest for which focus metric values are determined, such as a location of one or more regions of interest and/or a size of one or more regions of interest. Optionally, the one or more parameters are set at step 212 based on a type of a medical procedure. For example, one or more parameters may be set to a first set of values for a laparoscopic procedure (e.g., a range of focal plane settings associated with a longer working distance) and to a second set of values for an arthroscopic procedure (e.g., a range of focal plane settings associated with a shorter working distance).

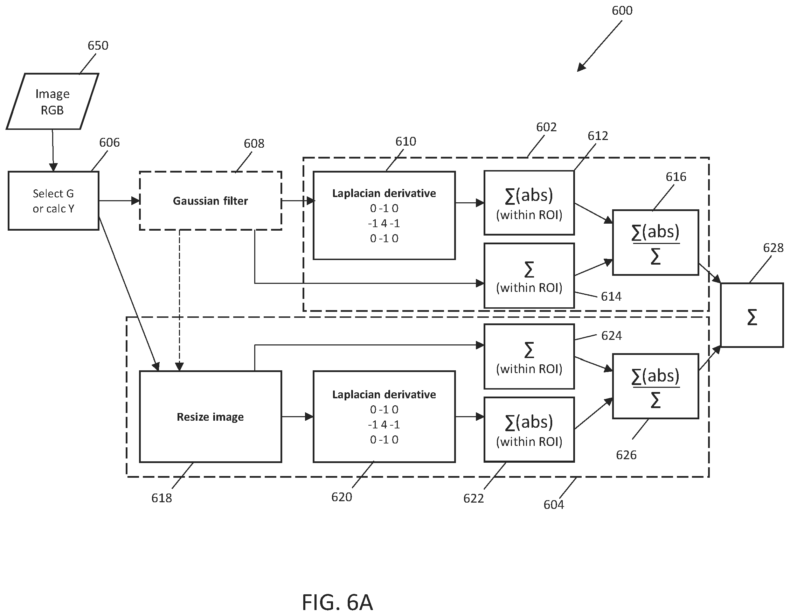

As noted above, step 204 includes determining focus metric values using multiple different focus metrics. FIG. 6A illustrates an example of a method 600 for determining a focus metric value based on two focus metrics. In the example of FIG. 6A, a first focus metric 602 operates on image data for the original image scale, and a second focus metric 604 operates on image data for a resized image. In operating on resized image data, focus metric 604 is more sensitive to contrast across a larger area than focus metric 602. Focus metric 602 is associated with more localized contrast than focus metric 604. Focus metric 604 may provide better information for determining how to adjust focal plane settings (in step 204 of method 200) when further from an optimal focal plane setting, whereas focus metric 602 may provide better information for determining how to adjust focal plane settings when closer to the optimal focal plane setting.

Method 600 may be performed at step 204 of method 200 and may be performed for each image received at step 202 of method 200. At step 606, a set of image data on which a focus metric value is to be calculated is determined from an image 650. The set of image data may be determined by converting the color image data of the image 650 to a pixel-by-pixel brightness estimate. For example, RGB values may be converted to luminance values (i.e., a luminance value for each pixel of the image 650). Alternatively, the set of image data determined at step 606 may be the green channel of the image 650 (i.e., the green values for each pixel of an RGB image 650), which in medical imaging may be a good approximation of luminance (and, thus, avoids the computation cost of computing the luminance channel). Optionally, at step 608, a gaussian filter is applied to the luminance or green image data to filter out high-frequency noise.

Computing a value for the first focus metric 602 includes, at step 610, determining a Laplacian derivative for each pixel of the filtered or non-filtered image data. Determining the Laplacian derivative may include applying a 3x3 kernel that calculates, for each pixel, a difference between the pixel and its neighbors. In the illustrated example, the Laplacian derivative function multiplies a value of a given pixel by four and subtracts the values of the neighboring pixels to the sides of the given pixel. At step 612, the sum of the absolute values of the Laplacian derivatives is determined. The sum may be determined for an entire image or may be determined for one or more regions of interest. At step 614, the values of the filtered or non-filtered image data are summed (for the entire image or for one or more regions of interest). At step 616, the sum of the Laplacian derivative values is normalized by the sum determined at step 614. The resulting value is the focus metric value for the first focus metric 602.

Computing a value for the second focus metric 604 includes, at step 618, determining a resized image from the non-filtered image data from step 606 or the filtered image data from step 604. The resized image may be determined by selecting pixel values based on a desired resized image size. For example, where a resized image is half the size of the original image, the resized image may be determined by selecting the values for every other pixel of the original image. In some examples, determining a resized image includes averaging, or otherwise combining, pixel values. For example, where a resized image is half the size of the original image, a value for a given pixel of the resized image may be an average of neighboring pixels of the original image.

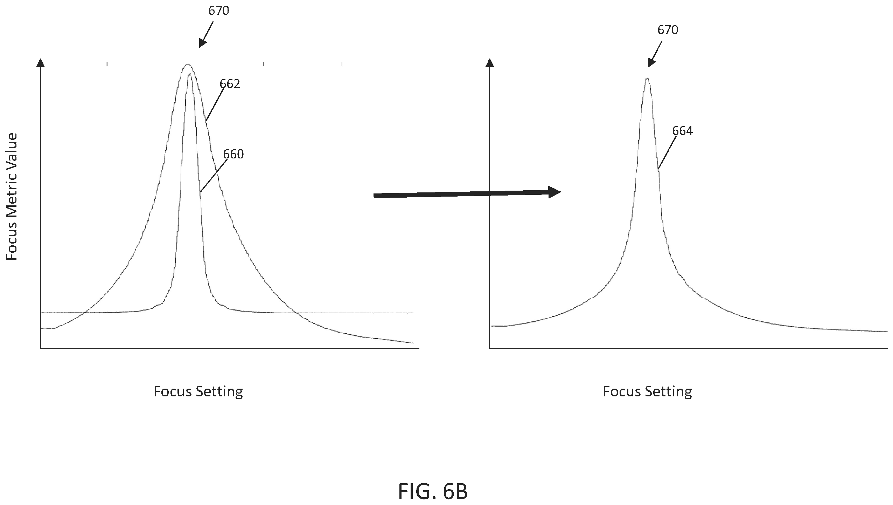

At step 620, a Laplacian derivative is determined for each pixel of the resized image determined at step 618. For example, the Laplacian derivative function may apply a 3x3 kernel that multiplies a value of a given pixel of the resized image by four and subtracts the values of the neighboring pixels to the sides of the given pixel. Steps 618 and 620 may be logically combined into a single operation such that a resized image is not generated. Rather, the kernel used for the Laplacian derivative may combine data from pixels further away from the central pixel (according to the desired resizing). For example, instead of the 3x3 kernel illustrated in FIG. 6B for the Laplacian derivative function used at step 620, a kernel may be a 9x9 kernel (or whatever scaling is desired) that subtracts values of pixels further away from the center pixel than the illustrated 3x3 kernel does. The kernel may be square or rectangular and may be any size.

Steps 622, 624, and 626 sum and normalize the values from step 620 and are analogous to steps 612, 614, and 616, respectively. Step 626 results in a focus metric value for the second focus metric 604. The focus metric values determined at steps 626 and 616 may be intermediate focus metric values that are used to calculate a final focus metric value at optional step 628. Step 628 may include the focus metric value associated with the first focus metric 602 and the focus metric value associated with the second focus metric 604. The focus metric values may be combined in various ways, such as via summing, averaging, or applying a weighted average. The resulting focus metric value is a function of the two different focus metrics-focus metric 602 and focus metric 604. The focus metric value determined at step 628 may be used for determining a focal plane setting adjustment in step 204. Alternatively, the focus metric values determined at steps 626 and 616 may not be combined, and one or both of the focus metric values determined at steps 626 and 616 may be used for determining a focal plane setting adjustment in step 204.

Optionally, instead of computing a focus metric value for the first focus metric 602 and a focus metric value for the second focus metric 604, the first and second focus metrics can be implemented in a single kernel that is applied once to each pixel of the image (or to each pixel of a region of interest of the image). In other words, the kernel from step 610 and the kernel from step 620 can be combined into a combined kernel. The values computed using the combined kernel can then be summed and normalized according to steps 612 and 614. The resulting value is a function of the first and second focus metrics but may be less computationally intensive to compute than computing intermediate focus metric values using each focus metric.

Focus metric 602 and focus metric 604 are merely illustrative examples of different focus metrics that may be used in method 200 of FIG. 2. Other examples of focus metrics that may be used include focus metrics that use different kernel sizes. A focus metric that uses a smaller kernel size (e.g., a 3x3 kernel as illustrated in FIG. 6A) is associated with more localized contrast than a focus metric that uses a larger kernel size (e.g., a 9x9 kernel) since the larger kernel size incorporates information from pixels that are further away relative to the smaller kernel size.

Optionally, which of a plurality of focus metrics are used for calculating a focus metric value for a given image may depend on one or more features in the image. For example, an image may be analyzed to determine a presence of one or more sharp objects and a size of the one or more sharp objects, and a focus metric may be selected based on the presence and/or size of the one or more sharp objects. If the size of the sharp objects in an image is relatively small (e.g., below a threshold value), a focus metric that uses a smaller kernel size may be used, and if the size of the sharp objects in an image is relatively large (e.g., above a threshold value), a focus metric that uses a larger kernel size may be used. Optionally, the size of sharp objects in an image may be determined using a machine learning model trained to detect sharp objects. In some examples, an estimate of the size of sharp objects in an image is determined by determining the standard variance for a focus metric for each pixel within a region of interest.

While method 600 is described above with reference to using a Laplacian derivative focus metrics, it will be readily understood by a person having ordinary skill in the art that other algorithms can be used to compute the focus metrics. For example, Tenengrad (Gradient Magnitude) uses the gradient magnitude (e.g., generated via a Sobel filter) to quantify sharpness. Variance of the image computes the variance of the grayscale image or its gradient. Brenner's Focus Measure uses the square difference with adjacent pixels. Fast Fourier Transform (FFT) High-Frequency Energy computes FFT and sum magnitude of high-frequency components. Sum of Modified Laplacian enhances the Laplacian by combining horizontal and vertical second derivatives. Wavelet-Based Sharpness decomposes an image using wavelets and uses the detail coefficients for sharpness. Perceptual Blur Metric (PBM) simulates human perception of blur by comparing edges in original and blurred versions of an image.

FIG. 6B illustrates an exemplary relationship between focus metric values provided by focus metric 602, focus metric values provided by focus metric 604, and focus metric values provided by a combination of focus metric 602 and focus metric 604. Curve 660 illustrates focus metric values for different focal plane settings that may be determined using focus metric 602. Curve 662 illustrates focus metric values for different focal plane settings that may be determined using focus metric 604. Focus metric 602 provides a more pronounced response (more extreme slope) near the maximum focus metric value 670 than focus metric 604, and thus, may be more suitable for use in adjusting focal plane settings when near the maximum focus metric value 670. However, focus metric 602 provides relatively little response (relatively small slope) further from the maximum focus metric value 670 such that it may be difficult to determine whether a maximum focus metric value has been reached and/or in which direction to adjust focal plane settings to reach the maximum focus metric value. In contrast, focus metric 604 provides a greater response (greater slope) further from the maximum focus metric value 670 than the first focus metric and, therefore, may be more useful for determining adjustments in focal plane settings when further away from the optimal focal plane setting. However, the response provided by the second focus metric 604 is more muted (smaller slope values) near the maximum focus metric value 670 than the response provided by the first focus metric value 602, which may increase the amount of time it takes to achieve the focal plane setting associated with the maximum focus metric value 670. The focus metric value determined at step 628 of method 600 is illustrated by curve 664. Curve 664 combines the greater response further away from the maximum focus metric value 670 provided by focus metric 604 with the greater response closer to the maximum focus metric value 670 provided by focus metric 602. Thus, determining a focus metric values using a combination of focus metric 602 and focus metric 604 can enable faster automatic focusing relative to using just focus metric 602 or just focus metric 604.

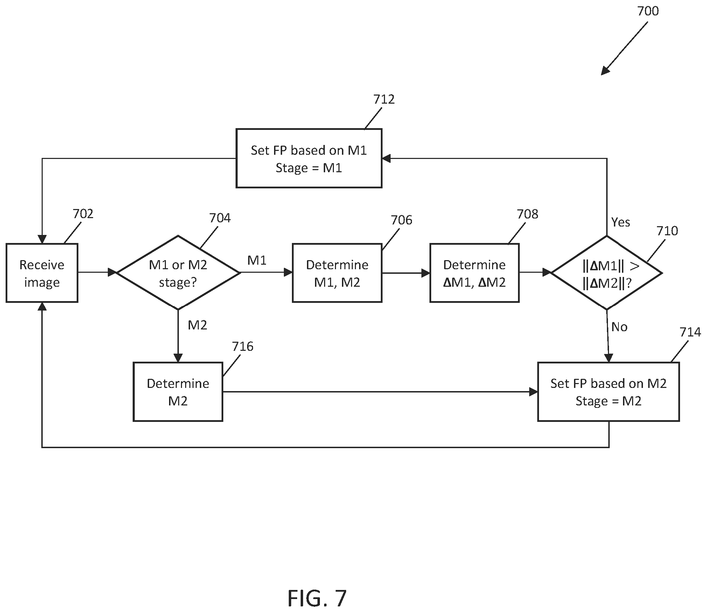

Instead of calculating a focus metric value using a combination of focus metrics, such as described above with respect to method 600, focus metric values for different focus metrics may be used at different stages of an automatic focusing process. For example, with reference to FIG. 6B, focus metric values calculated using focus metric 604 may be used for adjusting focal plane settings when further away from the maximum focus metric value 670 and focus metric values calculated using focus metric 602 may be used for adjusting focal plane settings when closer to the maximum focus metric value 670. FIG. 7 illustrates an exemplary method 700 for using different focus metrics at different stages of an automatic focusing process. Method 700 may be used in step 204 of method 200 of FIG. 2.

At step 702, an image is received in accordance with step 202 of method 200. At step 704, a determination is made whether a current stage of the automatic focusing process is a first focus metric stage or a second focus metric stage. When method 700 first begins, the stage may be initialized to the first focus metric stage, with the first focus metric being associated with greater response further from the maximum focus metric (e.g., focus metric 604 of FIG. 6A). If the current stage is the first focus metric stage ("M1" at step 704), then at step 706, a first focus metric value is determined for the image received at step 702 based on a first focus metric, and a second focus metric value is determined for the image based on a second focus metric. The second focus metric may be associated with a greater response closer to the maximum focus metric (e.g., focus metric 602 of FIG. 6A).

At step 708, a change in the respective focus metric value over time (i.e., relative to the focus metric value determined in previous iterations of method 700) is determined for each focus metric. According to some examples, step 708 can be understood as determining the slopes of curves 660 and 662 at a given focal plane setting based on focus metric values determined for the image received at step 702 and one or more previously received images. At step 710, a determination is made whether an absolute value of the change for the first focus metric value is greater than the absolute value of the change for the second focus metric value. Step 710 can be understood as determining which focus metric is providing a better response. If the first focus metric is providing the better response ("yes" at step 710), then at step 712, the first focus metric value is used to set a focal plane setting for a subsequent image. If, on the other hand, the second focus metric is providing a better response ("no" at step 710), then at step 714, the second focus metric is used to set the focal plane setting for the next response. Since the second focus metric is used to set the focal plane setting, the stage is the second focus metric stage. Method 700 then returns to step 702 for processing a next image.

Returning to step 704, if the determination is made that the stage is the second focus metric stage ("M2" at step 704), then a focus metric value is determined for the image received at step 702 using the second focus metric. Method 700 then moves to step 716, in which the second focus metric is used to set the focal plane setting for the next response. Thus, method 700 switches from using the first focus metric in an earlier stage of the automatic focusing process to using the second focus metric in a later stage of the automatic focusing process. The first focus metric may be used until the second focus metric provides a better response than the first focus metric.

Optionally, if use of the second focus metric does not result in finding the optimal focal plane setting (e.g., a sufficiently high peak is not found), method 700 may include switching back to using the first focus metric, such as by setting the stage to the first focus metric stage and following the "M1" branch of FIG. 7.

Instead of switching between use of different focus metrics based on which provides a better response, switching between use of different focus metrics may be based on a fixed step in a binary search. For example, a first focus metric may be used for a predetermined number of images and then the focus metric may be switched to a second focus metric. Alternatively, the first focus metric may be used until the change in the first focus metric values over time fall below a threshold. The threshold may be an absolute threshold or a relative threshold.

An automatic focusing process may include displaying images captured using the different focal plane settings used in the automatic focusing process. As such, a user may observe changes in focus as the system searches for the optimal focal plane setting. In some examples, automatic focusing operates on images that are not displayed to a user such that a user does not see any change in focus until the optimal focal plane setting is determined, at which point the user may see a step change to the optimal focal plane setting. For example, a frame rate of a camera, such as camera 104, may be sufficiently high that video of sufficiently high frame rate can be displayed to a user without displaying all of the frames captured by the camera (i.e., the video display frame rate is lower than the camera frame rate). Frames that are not displayed may be used for determining focal plane setting adjustment, such as in step 204 of FIG. 2. The focal plane setting of the camera may be alternated between a focal plane setting used for capturing images that are displayed and focal plane settings used for capturing images for performing automatic focusing. For example, with reference to FIG. 5, the camera 504 may capture images at sufficiently high frame rate and the lens assembly 504 may have sufficiently fast response time that the focal plane setting may be switched every other frame (or any other suitable fraction of the frame rate of the camera 504) between a focal setting used for displaying frames and focal plane settings used for automatic focusing.

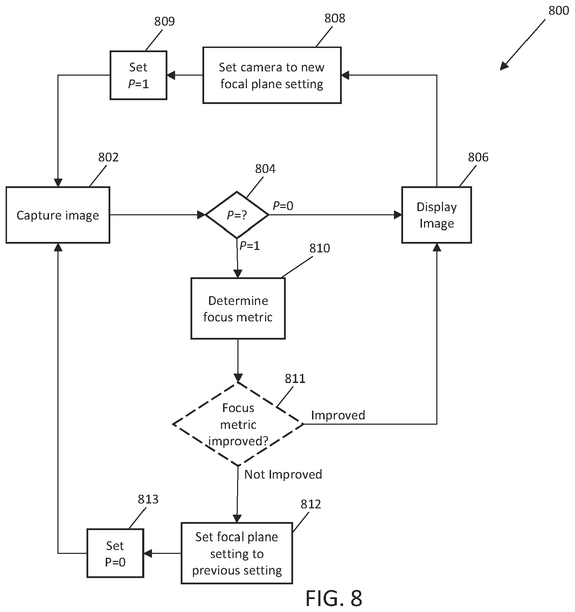

FIG. 8 illustrates an exemplary method 800 for performing automatic focusing using images that are not displayed. Method 800 can be used in combination with one or more steps of method 200. Method 800 tests focal plane settings on non-displayed images and maintains a given focal plane setting for displaying images until a better focal plane setting is found. Method 800 may switch between focal plane settings used for test images and focal plane settings used for displaying images according to a predefined switching sequence, such as every other image being used for display or every third image being used for display, and the other images used for focal plane setting testing.

At step 802, an image is captured, such as by camera 104 of FIG. 1. At step 804, a determination is made whether the image should be displayed or should be analyzed for improved focus by checking whether a current value of a parameter P is set to a predetermined value associated with displaying the image. For example, at the beginning of an automatic focusing process, P may be set to a value of 0, and step 804 for a first image captured at the start of the automatic focusing process results in a determination that P is equal to 0. If *P* is set to a predetermined value associated with display the image (e.g., *P*=0), the image is displayed at step 806.

At step 808, the focal plane setting of the camera is set to a new focal plane setting in accordance with an automatic focusing process (e.g., step 204 of method 200). At step 809, the parameter P is switched to a predetermined value associated with not displaying an image (e.g., *P* is set to 1). Method 800 returns to step 802 in which a new image is captured using the focal plane setting set at step 808. At step 804, the determination is made that parameter P is set to a predetermined value associated with not displaying the image (e.g., *P* is set to 1). Then, at step 810, a focus metric is determined for the new image according to the principles described herein.

Optionally, at step 811, the focus metric is compared to a focus metric of an image associated with a previously set focal plane setting and a determination is made whether the focus metric has improved. If the focus metric has improved ("yes"), the image may be displayed at step 806 and method 800 may continue with steps 808 and 809. Optionally, a filter may be used to provide a smoother transition from the previously displayed image.

If the focus metric has not improved as determined at step 811, or if step 811 is not performed, method 800 may continue to step 812 in which the focal plane setting is set to a previous setting associated with displaying images (e.g., the focal plane setting used for capturing the image previously displayed at step 806). At step 813, parameter P is set to a predetermined value associated with displaying an image (e.g., *P* is set to 0). Method 200 then returns to step 802 for capturing a next image. Method 800 may continue in this fashion until an optimal focal plane setting is determined (e.g., step 206 of method 200), at which point the focal plane setting may be set to the optimal focal plane setting and images captured at the optimal focal plane setting may be displayed. The automatic focusing process may end or may continue until manually stopped.

Method 800 switches between displayable and non-displayable focal plane settings (e.g., between each image). However, this is merely exemplary. In examples in which the frame rate of the camera is sufficiently high, the automatic focusing process may utilize more frames than are displayed. For example, frames may be displayed at a quarter of the frame rate at which images are captured such that one out of every four images is displayed and an automatic focusing process may utilize the other three out of every four frames for searching for the optimal focal plane setting. This may speed up the automatic focusing process, reducing the amount of time it takes to focus the camera.

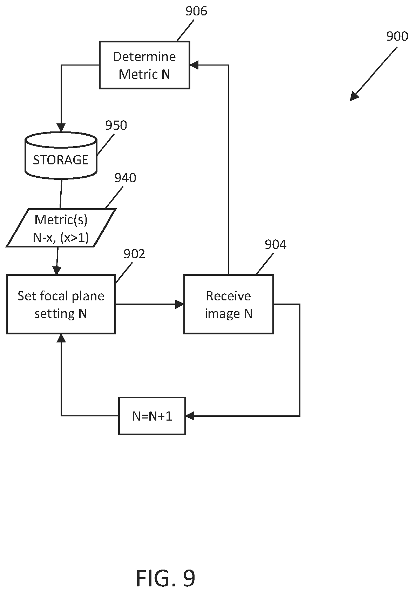

Another approach for speeding up the automatic focusing process is illustrated in FIG. 9, which includes a method 900 for automatic focusing using branch prediction. Method 900 may be used for step 204 of FIG. 2 and may be used instead of, or in combination with, method 600 of FIG. 6, method 700 of FIG. 7, and/or method 800 of FIG. 8. Method 900 includes changing the focal plane setting before knowing the focus metric value for a given focal plane setting. Method 900 can be useful in examples in which multiple images are captured in the delay between when an image is captured and when that image is processed for determining a focus metric, such as due to the latency in the imaging system. Rather than waiting for a focus metric to be determined for a given focal plane setting before changing the focal plane setting, method 900 can change the focal plane setting preemptively, such as by predicting a focal plane setting that will improve the focus metric. Once the focus metric for an image captured at a given focal plane setting is generated, a determination is made whether the preemptive change to the focal plane setting was correct. If the preemptive change to the focal plane setting was correct, then the autofocus process has gained time relative to a process that waits for the determination of the focus metric before changing focal plane setting because the focal plane setting has already been changed and an image has already been captured at that focal plane setting. If the preemptive change to the focal plane setting was not correct, then the preemptive change to the focal plane setting is discarded and the focal plane setting is changed according to the focus metric. In this case, no time is lost relative to a process that waits on the focus metric before changing the focal plane setting. Thus, the branch prediction approach of method 900 can save time in instances in which the preemptive change to the focal plane setting is correct and does not lose time (relative to a process that waits for the focus metric before changing the focal plane setting) in instances in which the preemptive change is not correct.

Method 900 includes, at step 902, setting a focal plane setting N to a value determined based on one or more focus metric values 940 calculated for one or more images captured using one or more previously set focal plane settings. The focus metric values 940 may be obtained from a memory 950, such as a buffer, that stores any number of previously determined focus metric values. The focus metric values can include values determined for images captured prior to the most recently captured image. For example, where the most recently captured image is image N-1, the focal plane setting may be determined based on focus metric values computed for images N-2, N-3, N-4, etc. Step 902 may include predicting what a focus metric value will be for image N-1 and determining focal plane setting N based on the prediction. For example, where the focal plane settings used to capture the images N-4, N-3, and N-2 are increasing and the associated focus metric values are increasing, step 902 may include predicting that the focus metric value for image N-1 will be greater than the focus metric values for image N-2 and determining that the focal plane setting N should be a given increment greater than the focal plane setting for image N-1. In some examples, the value for the focal plane setting N may be selected without any assessment of the likelihood that the value will improve the focus metric. Rather, the selection of the value for the focal plane setting N may be somewhat arbitrary. For example, the value for the focal plane setting N may simply be the next higher or next lower focal plane setting increment regardless of the trend of previous focus metric values.

Method 900 continues with step 904 in which an image is captured using the focal plane setting set at step 902. Method 900 then returns to step 902 in which a focal plane setting is determined for a next image. In parallel to this next instance of step 902, method 900 includes determining a focus metric value for image N at step 906. Once the focus metric value for image N is determined, the focus metric value may be stored in the memory 950. Determining the focus metric value for image N is asynchronous with steps 902 and 904 in the sense that the focus metric value for image N may not be computed in time for setting a focal plane setting for image N+1. Therefore, a focus metric value may not be determined for an image captured using a first focal plane setting until after the focal plane setting has been changed to another focal plane setting.

As noted above, a focal plane setting adjustment from a current focal plane setting to a next focal plane setting may be determined at step 902 based on a prediction associated with an improvement in focus metric value from a previously set focal plane setting to the current focal plane setting or may be a somewhat arbitrary selection that is not based on any assessment of likelihood that the adjustment is correct. Such a prediction may not ultimately prove correct. For example, a prediction for a focus metric for an image X that is used in setting focal plane setting X+1 at step 902 may turn out to not match with the focus metric value calculated for image X. Step 902 for focal plane setting X+2 may include comparing the focus metric value calculated for image X at step 906 with the prediction for the focus metric value for image X and, in response to the prediction being incorrect (e.g., the focus metric value reduced when it was predicted to increase), step 902 may include returning to a previously set focal plane setting, such as the focal plane setting for image X, or to a focal plane setting determined based on the focus metric value for image X. Where the delay between focal plane settings and focus metric values calculations encompasses the capture of multiple images, step 902 may include returning to a focal plane setting corresponding to an image of several iterations earlier in time.

Method 900 may include repeatedly reevaluating focal plane settings based on a latest set of focus metric values. Where a given set of focal plane setting adjustments is determined to deviate sufficiently from a focal plane setting adjustment that would have been done had the focus metric values been known, step 902 may include reverting to a previous focal plane setting or a focal plane setting determined based on the latest set of focus metric values.

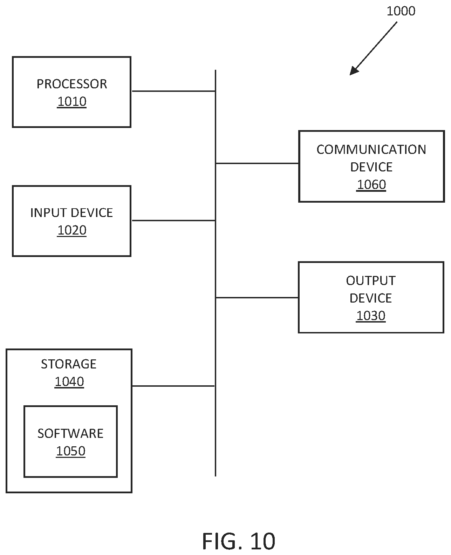

FIG. 10 illustrates an example of a computing system 1000 that can be used for one or more components of system 100 of FIG. 1, such as one or more of light source 108, camera control unit 120, camera 104, and image processing unit 122. System 1000 can be a computer connected to a network, such as one or more networks of a hospital, including a local area network within a room of a medical facility and a network linking different portions of the medical facility. System 1000 can be a client or a server. System 1000 can be any suitable type of processor-based system, such as a personal computer, workstation, server, handheld computing device (portable electronic device) such as a phone or tablet, or dedicated device. System 1000 can include, for example, one or more of input device 1020, output device 1030, one or more processors 1010, storage 1040, and communication device 1060. Input device 1020 and output device 1030 can generally correspond to those described above and can either be connectable or integrated with the computer.

Input device 1020 can be any suitable device that provides input, such as a touch screen, keyboard or keypad, mouse, gesture recognition component of a virtual/augmented reality system, or voice-recognition device. Output device 1030 can be or include any suitable device that provides output, such as a display, touch screen, haptics device, virtual/augmented reality display, or speaker.

Storage 1040 can be any suitable device that provides storage, such as an electrical, magnetic, or optical memory including a RAM, cache, hard drive, removable storage disk, or other non-transitory computer-readable medium. Communication device 1060 can include any suitable device capable of transmitting and receiving signals over a network, such as a network interface chip or device. The components of the computing system 1000 can be connected in any suitable manner, such as via a physical bus or wirelessly.

Processor(s) 1010 can be any suitable processor or combination of processors, including any of, or any combination of, a CPU, an FPGA, a GPU, and an ASIC. Software 1050, which can be stored in storage 1040 and executed by one or more processors 1010, can include, for example, the programming that embodies the functionality or portions of the functionality of the present disclosure (e.g., as embodied in the devices as described above), such as programming for performing one or more steps of method 200 of FIG. 2, method 600 of FIG. 6, method 700 of FIG. 7, method 800 of FIG. 8, and/or method 900 of FIG. 9.

Software 1050 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1040, that can contain or store programming for use by or in connection with an instruction execution system, apparatus, or device.

Software 1050 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport computer-readable medium can include, but is not limited to, an electronic, magnetic, optical, electromagnetic, or infrared wired or wireless propagation medium.

System 1000 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, Ethernet cables, fiber optic cables, DSL, or telephone lines.

System 1000 can implement any operating system suitable for operating on the network. Software 1050 can be written in any suitable programming language, such as C, C++, Java, or Python. In various examples, application software embodying the functionality of the present disclosure can be deployed in different configurations, such as in a client/server arrangement or through a web browser as a web-based application or web service, for example.

The following numbered illustrative embodiments are based on the disclosure of the present description. They provide explicit support for various combinations of features. Unless technically incompatible, features from different embodiments may be combined. Embodiment 1: A method for automatically focusing a medical camera comprising: receiving a plurality of images captured using different focal plane settings of the medical camera; while receiving the plurality of images, determining focus metric values for at least some of the plurality of images using a plurality of different focus metrics and setting the different focal plane settings based on the determined focus metric values; determining an optimal focal plane setting based on the focus metric values; and ceasing to set different focal plane settings such that images are captured using the optimal focal plane setting.
Embodiment 2: The method of embodiment 1, wherein determining the focus metric values for at least some of the plurality of images comprises determining the focus metric values using the plurality of focus metrics for each image of the at least some of the plurality of images.
Embodiment 3: The method of embodiment 1 or embodiment 2, wherein a focus metric value for a given image is determined based on a value associated with a first focus metric and a value associated with a second focus metric.
Embodiment 4: The method of any of the preceding embodiments, wherein determining the focus metric values for at least some of the plurality of images comprises using a first focus metric for a first set of images of the plurality of images and using a second focus metric but not the first focus metric for a second set of images of the plurality of images.
Embodiment 5: The method of any of the preceding embodiments, wherein the focus metric values are determined for at least one region of interest of the plurality of images.
Embodiment 6: The method of embodiment 5, comprising determining the at least one region of interest by analyzing at least one image of the plurality of images using a machine learning model.
Embodiment 7: The method of embodiment 5 or embodiment 6, comprising displaying at least one image of the plurality of images with an indication of the at least one region of interest.
Embodiment 8: The method of any of embodiments 5-7, wherein the focus metric values are determined for multiple regions of interest of the plurality of images.
Embodiment 9: The method of any of the preceding embodiments, wherein a first focus metric of the plurality of focus metrics is associated with more localized contrast than a second focus metric of the plurality of focus metrics.
Embodiment 10: The method of any of the preceding embodiments, wherein the first focus metric is associated with a smaller kernel size than the second focus metric.
Embodiment 11: The method of any of the preceding embodiments, comprising displaying a subset of images of the plurality of images, wherein at least one of the focal plane settings is set based on at least one focus metric value determined for at least one image that is not displayed.
Embodiment 12: The method of embodiment 11, comprising switching the focal plane of the medical camera between a focal plane setting used for the subset of images that are displayed and focal plane settings that are set based on focus metric values determined for images that are not displayed.
Embodiment 13: The method of any of the preceding embodiments, wherein setting the different focal plane settings based on the determined focus metric values comprises: adjusting the focal plane of the medical camera to a first focal plane setting based on a focus metric value determined for an image of the plurality of images associated with a previously set focal plane setting; prior to determining the focus metric value for an image associated with the first focal plane setting, adjusting the focal plane of the medical camera from the first focal plane setting to a second focal plane setting; and after adjusting the focal plane of the medical camera from the first focal plane setting to the second focal plane setting, determining at least one of the focus metric values for the image associated with the first focal plane setting.
Embodiment 14: The method of any of the preceding embodiments, wherein the optimal focal plane setting is determined in response to a user request to focus the medical camera.
Embodiment 15: The method of any of the preceding embodiments, wherein setting the different focal plane settings comprises controlling a liquid lens of the medical camera.
Embodiment 16: The method of any of the preceding embodiments, comprising receiving a request to focus the medical camera, and in response to receiving the request to focus the medical camera, setting at least one parameter used for determining the optimal focal plane setting to a predetermined value associated with a type of medical procedure.
Embodiment 17: The method of embodiment 16, wherein the at least one parameter comprises a parameter associated with defining a region of interest, a parameter associated with an initial focal plane setting, or a parameter associated with defining a range of focal plane settings.
Embodiment 18: The method of any of the preceding embodiments, wherein setting the different focal plane settings based on the determined focus metric values comprises skipping intermediate focal plane settings when changing from a first focal plane setting to a second focal plane setting.
Embodiment 19: The method of embodiment 18, wherein setting the different focal plane settings based on the determined focus metric values comprises changing from the second focal plane setting to one of the intermediate focal plane settings.
Embodiment 20: A method for automatically focusing a medical camera comprising: adjusting the focal plane of the medical camera to a first focal plane setting based on a focus metric value determined based on an image associated with a previously set focal plane setting; receiving an image captured at the first focal plane setting; adjusting the focal plane of the medical camera from the first focal plane setting to a second focal plane setting; and after adjusting the focal plane of the medical camera from the first focal plane setting to the second focal plane setting, determining at least one focus metric value based on the image captured at the first focal plane setting.
Embodiment 21: The method of embodiment 20, comprising, prior to adjusting the focal plane of the medical camera from the first focal plane setting to the second focal plane setting, selecting the second focal plane setting based on a prediction associated with an improvement in focus from the previously set focal plane setting to the first focal plane setting.
Embodiment 22: The method of embodiment 20 or embodiment 21, comprising computing the focus metric values based on a plurality of focus metrics.
Embodiment 23: The method of embodiment 22, wherein a first focus metric of the plurality of focus metrics is associated with more localized contrast than a second focus metric of the plurality of focus metrics.
Embodiment 24: The method of embodiment 23, wherein the first focus metric is associated with a smaller kernel size than the second focus metric.
Embodiment 25: The method of any of embodiments 20-24, wherein adjusting the focal plane comprises controlling a liquid lens of the medical camera.
Embodiment 26: The method of any of embodiments 20-25, comprising receiving a request to focus the medical camera, and in response to receiving the request to focus the medical camera, setting at least one parameter used for automatically focusing the camera to a predetermined value associated with a type of medical procedure.
Embodiment 27: The method of embodiment 26, wherein the at least one parameter comprises a parameter associated with defining a region of interest, a parameter associated with an initial focal plane setting, or a parameter associated with defining a range of focal plane settings.
Embodiment 28: A computing system for performing the method of any of the preceding embodiments.
embodiment 29: A computer program product comprising instructions for execution by one or more processors of a computing system for causing the computing system to perform the method of any of embodiments 1-27.

The foregoing description, for the purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the techniques and their practical applications. Others skilled in the art are thereby enabled to best utilize the techniques and various embodiments with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A method for automatically focusing a medical camera comprising:
receiving a plurality of images captured using different focal plane settings of the medical camera;
while receiving the plurality of images, determining focus metric values for at least some of the plurality of images using a plurality of different focus metrics and setting the different focal plane settings based on the determined focus metric values;
determining an optimal focal plane setting based on the focus metric values; and
ceasing to set different focal plane settings such that images are captured using the optimal focal plane setting.

2. The method of claim 1, wherein determining the focus metric values for at least some of the plurality of images comprises determining the focus metric values using the plurality of focus metrics for each image of the at least some of the plurality of images.

3. The method of claim 1 or claim 2, wherein a focus metric value for a given image is determined based on a value associated with a first focus metric and a value associated with a second focus metric.

4. The method of any of the preceding claims, wherein determining the focus metric values for at least some of the plurality of images comprises using a first focus metric for a first set of images of the plurality of images and using a second focus metric but not the first focus metric for a second set of images of the plurality of images.

5. The method of any of the preceding claims, wherein the focus metric values are determined for at least one region of interest of the plurality of images, wherein optionally the at least one region of interest is determined by analyzing at least one image of the plurality of images using a machine learning model, and/or wherein optionally at least one image of the plurality of images nis displayed with an indication of the at least one region of interest, and/or wherein optionally the focus metric values are determined for multiple regions of interest of the plurality of images.

6. The method of any of the preceding claims, wherein a first focus metric of the plurality of focus metrics is associated with more localized contrast than a second focus metric of the plurality of focus metrics, and/or wherein the first focus metric is associated with a smaller kernel size than the second focus metric.

7. The method of any of the preceding claims, comprising displaying a subset of images of the plurality of images, wherein at least one of the focal plane settings is set based on at least one focus metric value determined for at least one image that is not displayed.

8. The method of claim 7, comprising switching the focal plane of the medical camera between a focal plane setting used for the subset of images that are displayed and focal plane settings that are set based on focus metric values determined for images that are not displayed.

9. The method of any of the preceding claims, wherein setting the different focal plane settings based on the determined focus metric values comprises:
adjusting the focal plane of the medical camera to a first focal plane setting based on a focus metric value determined for an image of the plurality of images associated with a previously set focal plane setting;
prior to determining the focus metric value for an image associated with the first focal plane setting, adjusting the focal plane of the medical camera from the first focal plane setting to a second focal plane setting; and
after adjusting the focal plane of the medical camera from the first focal plane setting to the second focal plane setting, determining at least one of the focus metric values for the image associated with the first focal plane setting.

10. The method of any of the preceding claims, wherein the optimal focal plane setting is determined in response to a user request to focus the medical camera.

11. The method of any of the preceding claims, wherein setting the different focal plane settings comprises controlling a liquid lens of the medical camera.

12. The method of any of the preceding claims, comprising receiving a request to focus the medical camera, and in response to receiving the request to focus the medical camera, setting at least one parameter used for determining the optimal focal plane setting to a predetermined value associated with a type of medical procedure, wherein optionally the at least one parameter comprises a parameter associated with defining a region of interest, a parameter associated with an initial focal plane setting, or a parameter associated with defining a range of focal plane settings.

13. The method of any of the preceding claims, wherein setting the different focal plane settings based on the determined focus metric values comprises skipping intermediate focal plane settings when changing from a first focal plane setting to a second focal plane setting, wherein optionally setting the different focal plane settings based on the determined focus metric values comprises changing from the second focal plane setting to one of the intermediate focal plane settings.

14. A computing system for performing the method of any of the preceding claims.

15. A computer program product comprising instructions for execution by one or more processors of a computing system for causing the computing system to perform the method of any of claims 1-13.
